# EUROPEAN PATENT SPECIFICATION

(11) **EP 1 608 412 B1**
(45) Date of publication and mention of the grant of the patent: **02.01.2008**
(21) Application number: 04714794.7
(22) Date of filing: 26.02.2004
(51) Int. Cl.: A61K 51/04

(54) **RADIOLABELLED SUBSTRATES**
RADIOAKTIV MARKIERTE SUBSTRATE
SUBSTRATS RADIOMARQUES

(30) Priority: 28.02.2003 GB 0304681
(43) Date of publication of application: 28.12.2005
(73) Proprietor: GLAXO GROUP LIMITED, Greenford, Middlesex UB6 0NN (GB)
(72) Inventor: GEE, Antony David, GlaxoSmithKline, Cambridge, CambridgeshireCB2 2GG (GB)
(74) Representative: West, Vivien
(86) International application number: PCT/EP2004/002008
(87) International publication number: WO 2004/075926

(56) References cited:
- SCHINKEL ET AT: "P-glycoprotein in the blood brain barrier of mice influences the brain penetration and pharmacological activity of many drugs" J.CLIN.INVEST, vol. 97, no. 11, 1996, pages 2517-2524, XP002282704 cited in the application

## Description

The present invention relates to radiolabelled P-glycoprotein (P-gp) substrates, which are useful for the labelling and diagnostic imaging of P-gp functionality.

Noninvasive, nuclear imaging techniques can be used to obtain basic and diagnostic information about the physiology and biochemistry of living subjects, including experimental animals, normal humans and patients. These techniques rely on the use of imaging instruments that can detect radiation emitted from radiotracers administered to living subjects. The information obtained can be reconstructed to provide planar and tomographic images which reveal the distribution and/or concentration of the radiotracer as a function of time.

Positron emission tomography (PET) is a noninvasive imaging technique that offers the highest spatial and temporal resolution of all nuclear medicine imaging modalities and has the added advantage that it can allow for true quantitation of tracer concentrations in tissues. The technique involves the use of radiotracers, labelled with positron emitting radionuclides, that are designed to have *in vivo* properties which permit measurement of parameters regarding the physiology or biochemistry of a variety of processes in living tissue.

Compounds can be labelled with positron or gamma emitting radionuclides. The most commonly used positron emitting radionuclides are ¹⁵O, ¹³N, ¹¹C and ¹⁸F, which are accelerator produced and have half lifes of 2, 10, 20 and 110 minutes respectively. The most widely used gamma emmitting radionuclides are ¹⁸F, ^{99m}Tc, ²⁰¹TI and ¹²³I_{.}

P-glycoprotein (P-gp) is an ATP-driven transmembrane efflux pump located in, among other tissues, the blood brain barrier (Bradbury, 1993), kidneys, and testes (Sugawara, 1990). In humans, this protein is encoded by the MDR1 gene. Over the last decade, a large number of structurally diverse compounds have been shown to be transported out of cells by P-gp, leading to a much lower availabilty of these compounds in their intended tissues than would be expected from the physical properties of the compounds (Schinkel, Wagenaar et al., 1996) which only share the properties of being small (usually <2kDa) hydrophobic amphipathic molecules that are usually not negatively charged. Classes of small molecule therapeutics which are substrates of P-gp include anticancer, immunosuppressive, cardiac, antihistimine and a number of anti-infectives including agents effective against human immunodeficency virus (HIV). The activity of P-gp decreases the intracellular availability of a variety of anticancer drugs, leading to the development of resistance to them; the same appears to be true for HIV protease and non-nucleoside reverse transcriptase inhibitors (Fellay,J., Marolini,C., et. al 2002)

A number of PET and SPECT (single photon emission tomography) tracers have been developed to demonstrate the presence of P-gp in tissue, but none of these tracers are applied to drug development or currently used as routine clinical diagnostic tool (Del Vecchio, Ciarmiello et al., 2000;Hendrikse and Vaalburg, 2002;Levchenko, Mehta et al., 2000). Although these imaging tools have their utility, their sensitivity and therefore their scope for research purposes is limited. At most, a 2-3 fold increase of uptake in the P-gp expressing tissue (brain/tumour) is observed at the assumed 100% inhibition dose. This means that if small changes (e.g. <20%) in P-gp functionality suffice for co-treatment in for example tumour therapy, current imaging tools may not be sensitive enough to establish the change in P-gp functionality with sufficient confidence and may therefore not be suitable for establishing the required dose of P-gp inhibitor or competative substrate. The P-gp transport system is complex and poorly understood in man *in vivo* and highly sensitive radiotracers which could be used *in vivo* would be especially beneficial in elucidating P-gp's role in drug and toxin resistance, immunity, apoptosis or cell differentiation.

According to the present invention here is provided a radiolabelled compound of formula (I): 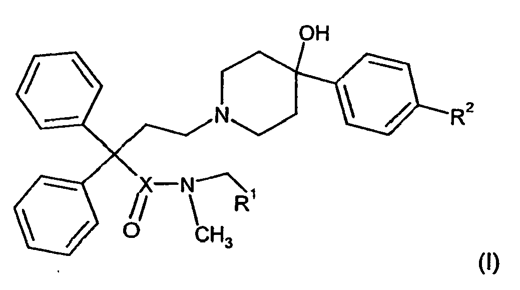 for use as a P-glycoprotein (P-gp) substrate, wherein:
X is ¹¹C or C;
R¹ is R³ when X is C or R¹ is hydrogen when X is ¹¹C;
R² is C₁₋₆alkyl, C₁₋₆alkoxy, N(Me)₂ or halogen when X is ¹¹C or when R¹ is R³ or R² is R³ or C₁₋₆alkyl, C₁₋₆alkoxy or N(Me)₂ labelled with R³ when X is C or when R¹ is hydrogen;
R³ is ¹¹C, ¹⁸F, ¹²³I, ¹²⁵I, ⁷⁶Br, ⁷⁷Br or cyclopentadienyl ^{99m}Tc;
or a pharmaceutically acceptable salt thereof.

Preferably R² is methyl, methoxy, N(Me)₂, F, Br, I or Cl.

More preferably X is C, R¹ is ¹¹C and R² is Cl.

The present invention also provides a radiopharmaceutical composition which comprises a compound of formula (I) and a pharmaceutically acceptable carrier or excipient.

One of the compounds of formula (I) is 4-(4-chlorophenyl)-4-hydroxy-N,N-dimethyl-alpha,alpha-diphenyl-1-piperidinebutyramide, more commonly known as loperamide (Imodium™), an opioid receptor agonist used in the treatment of diarrhoea (Galambos, Hersh et al., 1976), It is also a compound which interacts with and is transported by P-gp (Sadeque, Wandel et al., 2000;Schlnkel, Wagenaar, Mol, and van Deemter, 1996;Wandel, Kim et al., 2002).

The present invention also relates to a process for the preparation of [¹¹C-*N*-*methyl*]-4-(4-chlorophenyl)-4-hydroxy-N,N-dimethyl-alpha,alpha-diphenyl-1-piperidinebutyramide which comprises reacting 4-[4-(4-Chloro-phenyl)-4-hydroxy-piperidin-1-yl]-*N*-methyl-2,2-diphenyl-butyramide, the desmethyl loperamide precursor with [¹¹C]Mel in the presence of a base, such as potassium hydroxide and an inert solvent, such as dimethyl sulfoxide at a temperature between room temperature and solvent reflux temperature, preferably about 80°C; and isolating the product by high performance liquid chromatography (HPLC) and any remaining organic solvents removed by any conventional means.

Suitable radionuclides that may be incorporated in compounds of formula (I) include: ¹¹C, ¹⁸F, ^{99m}Tc, ¹²³I, ¹²⁵I, ⁷⁶Br, and ⁷⁷Br. The choice of radionuclide to be incorporated into compounds of formula (I) will depend on the specific analytical or pharmaceutical application. Therefore, for *in vitro* labelling of P-gp and for competition assays compounds that incorporate ¹²⁵I or ⁷⁷Br would be preferred. For diagnostic and investigative imaging agents, compounds that incorporate a radionuclide selected from ¹¹C, ¹⁸F, ^{99m}Tc, ¹²³I or ⁷⁶Br are preferred. Incorporation of a chelating radionuclide such as ^{99m} Tc may be useful in certain applications.

Radiolabelled analogues of loperamide may be used in clinical studies to evaluate the role of P-gp inhibitors in a variety of disease areas where P-glycoprotein is believed to play a role in therapy failure e.g. expression of P-gp on the luminal surfaces of the intestinal epithelial cells leading to diminishing adsorption of orally dosed compounds, efflux of HIV Protease inhibitors resulting in lowered bioavailability and delivery to tissues, or anti tumour therapy where the presence of P-glycprotein can lead to desensitisation of tumours against for example antineoplastics.

Scheme 1 represents the synthetic route towards cGMP quality desmethyl-loperamide

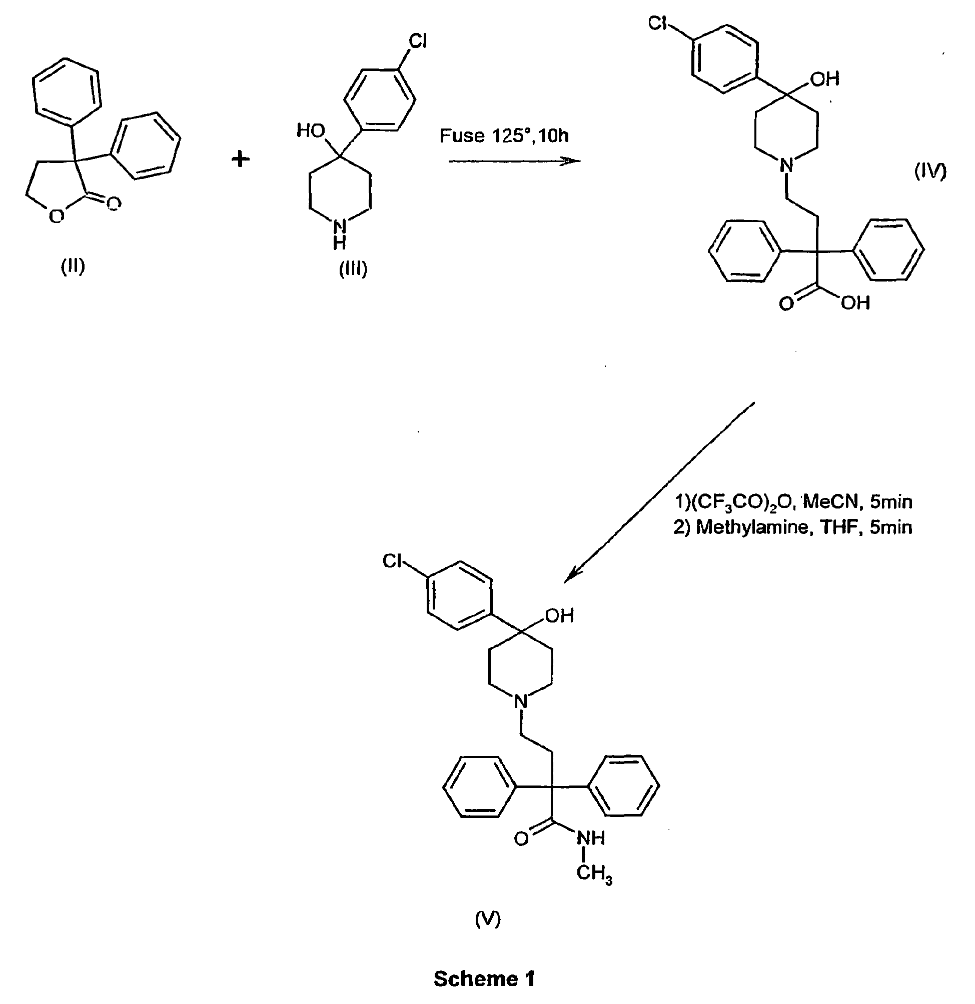

In this scheme, 3,3-Diphenyl-dihydro-furan-2-one (II) is fused with 4-(4-Chloro-phenyl)-piperidin-4-ol (III) giving 4-[4-(4-Chloro-phenyl)-4-hydroxy-piperidin-1-yl]-butyric acid (IV) which is converted into its N-methyl amide via in-situ formation of the mixed anhydride by reaction with trifluoroacetic anhydride, followed by treatment with methylamine to yield desmathyl loperamide (V).

The synthetic route for the synthesis of a compound of formula (I) labelled with [¹¹C] is shown in Scheme 2.

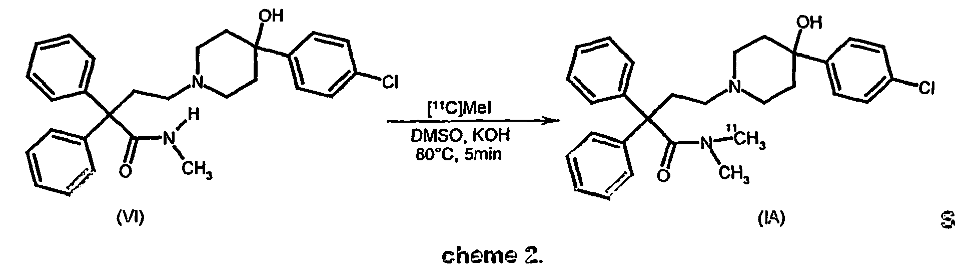

Typically, the desmethyl loperamide precursor is reacted with [¹¹C]Mel at 80°C for 5 min using DMSO as solvent and KOH as base. The product is obtained by reverse phase HPLC, and, following removal of organic solvents by means of *in vacuo* evaporation, is formulated in a sterile 0.9% saline solution.

One possible route to radio-fluorinated and radio-iodinated analogues of compounds of formula (I) is described in Scheme 3.

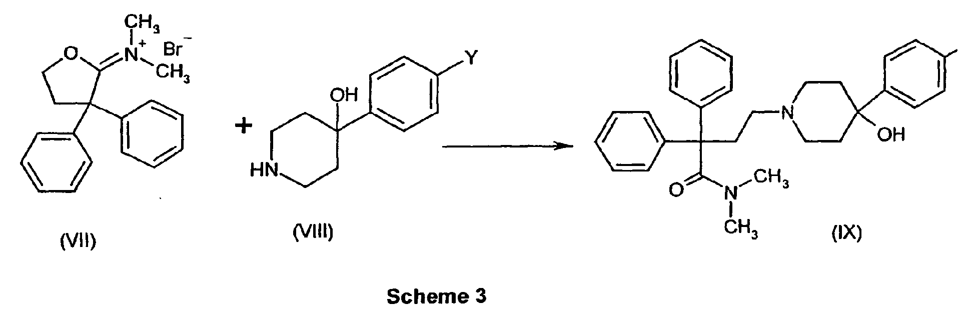

For the production of radiofluorinated products Y is an iodinium salt moiety or a triazene moiety from which the final product can be made through direct nucleophilic reaction with ¹⁸F-fluoride. Typically this would be performed in an organic solvent (DMF, DMSO, ACN) using kryptofix® 222 as a phase transfer agent. For the production of radioiodinated products Y is a trialkylstannyl moiety, a boronic acid moiety or a halogen moiety from which the radioiodinated analogue can be produced through a variety of nucleophilic or electrophilic reactions.

Alternatively, compounds of formula(I) can be labelled in the carbonyl (X) position using a Pd(0) mediated coupling reaction between the appropriate halogenated substrate, [¹¹C]CO, and the appropriate secondary amine.

The starting materials and other reagents are available commercially or can be synthesised by well-known and conventional methods.

### Biological Data

The animals (pig, Yorkshire landrace, 37.5kg, n=2) were scanned under terminal aneasthesia (ketamine induced isoflurane aneasthesia) on different days. For both animals an arterial cannula was inserted in one of the femoral arteries. [¹¹C-*N*-*methyl*]-4-(4-chlorophenyl)-4-hydroxy-N,N-dimethyl-alpha,alpha-diphenyl-1-piperidinebutyramide ([¹¹C]loperamide)was administered intravenously into the femoral vein, contralateral to the arterial cannula as a 1 min bolus. PET scanning and arterial blood sampling was commenced upon start of administration. Cyclosporin A (Sandimmune^{™}) was used as a competitive F-glycoprotein substrate (Twentyman, 1992). Under baseline conditions, little CNS peneration was observed for [¹¹C]loperamide with % injected dose /L staying below 1%. Intravenous administration of various doses of cyclosporin A, 30 min prior to injection of (I), led to a dose dependent increase of radioactive signal in the brain as measured with PET scanning. [¹⁵O]CO and [¹⁵O]H₂O were administered intravenously into pig under baseline conditions and following each intravenous dose of cyclosporin A to correct for changes in bloodvolume and changes in bloodflow. Maximum increase of CNS penetration of (I) for the brain as a whole was 7-fold and was observed at a dose of 40-50 mg/kg Cyclosporin A (CsA). Comparison of the rate of delivery K₁ of (I) with the K₁ of [¹⁵O]H₂O indicated that at this dose virtually all P-glycoprotein was inhibited. At doses of CsA>10mg/kg, the rate of metabolism of [¹¹C]loperamide was decreased from approx 50% parent to approx 80% parent at 60 min post administration. Cerebral bloodflow was marginally increased at the higher doses of CsA. These changes only contribute to a small extent to the large changes in signal that were observed.

### References

1. Bradbury,M.W., 1993. The blood-brain barrier. Exp.Physiol 78, 453-472.
2. Del Vecchio,S., Ciarmiello,A., Salvatore,M., 2000. Scintigraphic detection of multidrug resistance in cancer. Cancer Biother.Radiopharm. 15, 327-337.
3. Fellay,J., Marzolini,C., Meaden,E.R., Back,D.J., Buclin,T., Chave,J.P., Decosterd,L.A., Furrer,H., Opravil,M., Pantaleo,G., Retelska,D., Ruiz,L., Schinkel,A.H., Vernazza,P., Eap,C.B., Telenti,A., 2002. Response to antiretroviral treatment in HIV-1-infected individuals with allelic variants of the multidrug resistance transporter 1: a pharmacogenetics study. Lancet 359, 30-36.
4. Galambos,J.T., Hersh,T., Schroder,S., Wenger,J., 1976. Loperamide: a new antidiarrheal agent in the treatment of chronic diarrhea. Gastroenterology 70, 1026-1029.
5. Hendrikse,N., Vaalburg,W., 2002. Dynamics of multidrug resistance: P-glycoprotein analyses with positron emission tomography. Methods 27, 228.
6. Levchenko,A., Mehta,B.M., Lee,J.B., Humm,J.L., Augensen,F., Squire,O., Kothari,P.J., Finn,R.D., Leonard,E.F., Larson,S.M., 2000. Evaluation of 11C-colchicine for PET imaging of multiple drug resistance. J.Nucl Med. 41, 493-501.
7. Sadeque,A.J., Wandel,C., He,H., Shah,S., Wood,A.J., 2000. Increased drug delivery to the brain by P-glycoprotein inhibition. Clin.Pharmacol.Ther. 68, 231-237.
8. Schinkel,A.H., Wagenaar,E., Mol,C.A., van Deemter,L., 1996. P-glycoprotein in the blood-brain barrier of mice influences the brain penetration and pharmacological activity of many drugs. J.Clin.Invest 97, 2517-2524.
9. Sugawara,I., 1990. Expression and functions of P-glycoprotein (mdr1 gene product) in normal and malignant tissues. Acta Pathol.Jpn. 40, 545-553.
10. Twentyman,P.R., 1992. Cyclosporins as drug resistance modifiers. Biochem.Pharmacol. 43, 109-117.
11. Wandel,C., Kim,R., Wood,M., Wood,A., 2002. Interaction of morphine, fentanyl, sufentanil, alfentanil, and loperamide with the efflux drug transporter P-glycoprotein. Anesthesiology 96, 913-920.

## Claims

1. A radiolabelled compound for use as a P-glycoprotein (P-gp) substrate, according to formula (I): 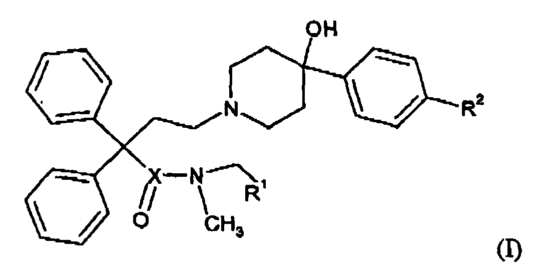 wherein:
X is ¹¹C or C;
R¹ is R³ when X is C, or R¹ is hydrogen when X is ¹¹C;
R² is C₁₋₆alkyl, C₁₋₆alkoxy, N(Me)₂ or halogen when X is ¹¹C or when R¹ is R³, or R² is R³ or C₁₋₆alkyl, C₁₋₆alkoxy or N(Me)₂ labelled with R³ when X is C or when R¹ is hydrogen;
R³ is ¹¹C, ¹⁸F_{,} ¹²³I ¹²⁵I, ⁷⁶Br, ⁷⁷Br or cyclopentadienyl ^{99m}Tc;
or a pharmaceutically acceptable salt thereof.

2. A compound according to claim 1, wherein R² is methyl, methoxy, N(Me)2, F, Br, I or Cl.

3. A compound according to claim 1 or 2, wherein R¹ is ¹¹C.

4. A compound according to any preceding claim, wherein X is C, R¹ is ¹¹C and R² is Cl.

5. A pharmaceutical composition which comprises a compound of formula (I) according to any preceding claim and a pharmaceutically acceptable carrier or excipient.

6. A process for the preparation of [¹¹C-*N*-*methyl*]-4-(4-chlorophenyl)-4-hydroxy-N,N-dimethyl-alpha,alpha-diphenyl-1-piperidinebutyramide which comprises reacting a compound of formula (VI) 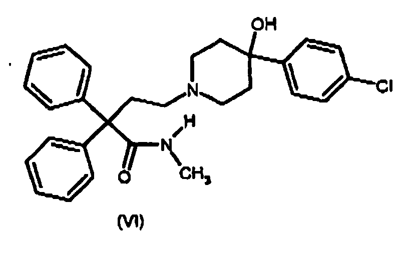 with [¹¹C]MeI and isolating the product.

## Patentansprüche

1. Radioaktiv markierte Verbindung gemäß Formel (I): 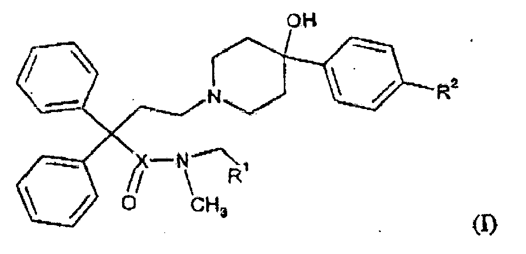 oder ein pharmazeutisch verträgliches Salz davon
zur Verwendung als P-Glycoprotein(P-gp)-Substrat, wobei:
X ¹¹C oder C ist;
R¹ R³ ist, wenn X C ist, oder R¹ Wasserstoff ist, wenn X ¹¹C ist;
R² C₁₋₆-Alkyl, C₁₋₆-Alkoxy, N(Me)₂ oder Halogen ist, wenn X ¹¹C ist oder wenn R¹ R³ ist, oder R² R³ oder mit R³ markiertes C₁₋₆-Alkyl, C₁₋₆-Alkoxy oder N(Me)₂ ist, wenn X C ist oder wenn R¹ Wasserstoff ist;
R^{3 11}C, ¹⁸F, ¹²³I, ¹²¹I, ⁷⁶Br, ⁷⁷Br oder Cyclopentadienyl-^{99m}Tc ist.

2. Verbindung gemäß Anspruch 1, wobei R² Methyl, Methoxy, N(Me)₂, F, Br, I oder Cl ist.

3. Verbindung gemäß Anspruch 1 oder 2, wobei R^{1 11}C ist.

4. Verbindung nach einem der vorangehenden Ansprüche, wobei X C ist, R^{1 11}C ist und R² Cl ist.

5. Arzneimittel, welches eine Verbindung der Formel (I) gemäß einem der vorangehenden Ansprüche und einen pharmazeutisch verträglichen Träger oder Exzipienten umfasst.

6. Verfahren zur Herstellung von [¹¹C-N-Methyl]-4-(4-chlorphenyl)-4-hydroxy-N,N-dimethyl-alpha,alpha-diphenyl-1-piperidinbutyramid, welches das Umsetzen einer Verbindung der Formel (VI) 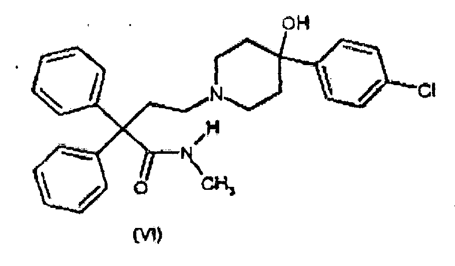 mit [¹¹C]MeI und Isolieren des Produkts umfasst.

## Revendications

1. Composé radiomarqué destiné à être utilisé comme substrat de glycoprotéine P (P-gp), répondant à la formule (I) : 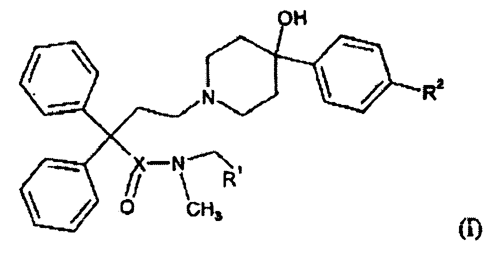 dans laquelle :
X représente ¹¹C ou C ;
R¹ représente un groupe R³ lorsque X représente C, ou bien R¹ représente un atome d'hydrogène lorsque X représente ¹¹C ;
R² représente un groupe alkyle en C₁ à C₆, alkoxy en C₁ à C₆, N(Me)₂ ou un atome d'halogène lorsque X représente ¹¹C ou lorsque R¹ représente un groupe R³, ou bien R² représente un groupe R³ ou alkyle en C₁ à C₆, alkoxy en C₁ à C₆ ou N(Me)₂ marqué avec R³ lorsque X représente C ou lorsque R¹ représente un atome d'hydrogène ;
R³ représente ¹¹C, ¹⁸F, ¹²³I, ¹²⁵I, ⁷⁶Br, ⁷⁷Br ou le cyclopentadiényle ^{99m}Tc ;
ou un de ses sels pharmaceutiquement acceptables.

2. Composé suivant la revendication 1, dans lequel R² représente un groupe méthyle, méthoxy, N(Me)₂, F, Br, I ou Cl.

3. Composé suivant la revendication 1 ou 2, dans lequel R¹ représente ¹¹C.

4. Composé suivant l'une quelconque des revendications précédentes, dans lequel X représente C, R¹ représente ¹¹C et R² représente un groupe Cl.

5. Composition pharmaceutique qui comprend un composé de formule (I) suivant l'une quelconque des revendications précédentes et un support ou excipient pharmaceutiquement acceptable.

6. Procédé pour la préparation de [¹¹C*-N-méthyl*]-4-(4-chlorophényl)-4-hydroxy-N,N-diméthyl-alpha,alpha-diphényl-1-pipéridinebutyramide qui comprend la réaction d'un composé de formule (VI) 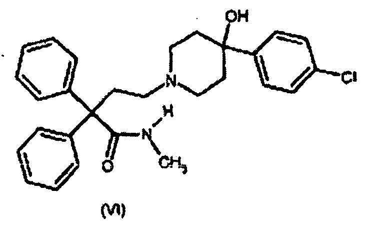 avec du [¹¹C]MeI et l'isolement du produit.
